# FASCICULE DE BREVET EUROPEEN

(11) **EP 2 086 440 B1**
(45) Date de publication et mention de la délivrance du brevet: **29.08.2012**
(21) Numéro de dépôt: 07848269.2
(22) Date de dépôt: 21.09.2007
(51) Int. Cl.: A61B 18/14

(54) **DISPOSITIF DE PINCE ÉLECTRO-CHIRURGICALE**
ELEKTROCHIRURGISCHE ZANGENVORRICHTUNG
ELECTROSURGICAL FORCEPS DEVICE

(30) Priorité: 27.09.2006 FR 0608473
(43) Date de publication de la demande: 12.08.2009
(73) Titulaire: Noury, Guillaume, 78670 Villennes sur Seine (FR)
(72) Inventeur: Noury, Guillaume, 78670 Villennes sur Seine (FR)
(74) Mandataire: CAPRI
(86) Numéro de dépôt international: PCT/FR2007/001540
(87) Numéro de publication internationale: WO 2008/037879

(56) Documents cités:
- WO-A-00/61014
- US-A1- 5 800 449
- US-A1- 6 030 409
- US-A1- 2004 102 772
- US-B2- 6 858 028

## Description

La présente invention concerne un dispositif de pince électro-chirurgicale.

Plus particulièrement, la présente invention concerne une pince électro-chirurgicale de thermofusion pour chirurgie ouverte et pour coeliochirurgie, c'est-à-dire une chirurgie dans laquelle la pince est introduite à l'intérieur du corps du patient à travers un tube ou trocart.

Les pinces électro-chirurgicales sont bien connues dans l'état de la technique, en particulier pour réaliser la thermofusion des tissus ou des vaisseaux dans le corps d'un patient. Lors de la thermofusion, l'énergie calorifique dissipée par un courant bipolaire de haute fréquence et de forte intensité appliquée sur une veine ou une artère transforme puis fusionne le collagène et l'élastine des tissus en formant une soudure permanente des parois vasculaires sans formation de caillot. La thermofusion permet de remplacer les autres procédés de ligature ou d'hémostase des vaisseaux aussi bien en chirurgie ouverte qu'en coeliochirurgie. Des applications de la thermofusion comprennent notamment les ablations totales ou partielles, telles que les colectomies, les cholécystectomies, l'hystérectomie, la thyroïdectomie, etc. La thermofusion permet aussi de réaliser la soudure de deux tissus entre eux. Pour réaliser une telle thermofusion, on utilise généralement des pinces bipolaires de thermofusion connectées à un générateur électro-chirurgical hautes fréquences.

Les pinces électro-chirurgicales existantes à ce jour présentent toutes des inconvénients. Ainsi, il existe des pinces chirurgicales dépourvues de moyens de coupe. Ces pinces présentent l'inconvénient qu'après thermofusion des tissus et/ou des vaisseaux à l'intérieur du corps, le chirurgien doit retirer la pince, pour venir ensuite couper par d'autres moyens les tissus qui ont été soudés thermiquement. Ceci impose deux prises consécutives (une première pour la thermofusion, une seconde pour la coupe) pour le chirurgien, avec la contrainte d'effectuer la seconde prise exactement au même endroit que la première prise. Par ailleurs, il a été proposé des pinces chirurgicales de thermofusion comportant des dispositifs de coupe intégrés. Ces pinces présentent toutefois un inconvénient majeur, en ce qu'elles sont à usage unique, c'est-à-dire qu'après chaque utilisation, la totalité du dispositif doit être jeté. En effet, les différentes parties mobiles de ces dispositifs de pince ne sont pas nettoyables, décontaminables et/ou stérilisables, de sorte qu'ils ne sont pas réutilisables. Le coût d'utilisation de ces pinces est donc très élevé. De plus, les pinces chirurgicales intégrant un dispositif de coupe sont réalisées de telle sorte que la pince se ferme pour réaliser la thermofusion lorsque l'utilisateur actionne la poignée et qu'elle est ouverte en position de repos de la poignée. Dans le cadre notamment d'une coeliochirurgie, le chirurgien doit donc actionner la poignée pour fermer la pince pour pouvoir l'introduire à travers le trocart et le tube à l'intérieur du corps. Il doit donc à la fois fermer sa main pour actionner la poignée et avancer le bras pour introduire la pince dans le corps. Ceci complique l'utilisation de la pince. La présence d'un dispositif de coupe dans une pince chirurgicale implique aussi dans les dispositifs existants que la pince ne peut pas tourner librement de manière infinie dans les deux sens autour de l'axe longitudinal du tube reliant la pince à la poignée. Ceci peut parfois imposer au chirurgien de tourner dans un sens puis dans un autre pour arriver à la position idéale dont il a besoin pour effectuer son opération. A nouveau, l'utilisation de la pince en devient compliquée. Les documents US-5 451227, US 2004/102772, US-6 030 409 et US-5 800 449 décrivent des dispositifs de l'art antérieur.

La présente invention a pour but de fournir une pince électro-chirurgicale qui ne reproduit pas les inconvénients susmentionnés.

En particulier, la présente invention a pour but de fournir une pince électro-chirurgicale qui soit réutilisable un nombre quelconque de fois.

La présente invention a également pour but de fournir une pince électro-chirurgicale qui puisse facilement être nettoyée, décontaminée et stérilisée après chaque utilisation.

La présente invention a encore pour but de fournir une pince électro-chirurgicale dans laquelle le dispositif de coupe et/ou les coiffes des mors de la pince peuvent être interçhangés après un nombre quelconque d'utilisations, éventuellement après chaque utilisation de la pince.

La présente invention a également pour but de fournir une pince chirurgicale de thermofusion dont le coût d'utilisation est fortement diminué, notamment par rapport aux dispositifs jetables à usage unique.

La présente invention a aussi pour but de fournir une pince chirurgicale de thermofusion plus simple, plus fiable, plus précise et plus sûre à utiliser.

La présente invention a donc pour objet un dispositif de pince électro-chirurgicale tel que décrit dans la revendication 1. Des modes de réalisation avantageux sont décrits dans les revendications dépendantes.

Ces caractéristiques et avantages et d'autres de la présente invention apparaîtront plus clairement au cours de la description détaillée suivante d'un mode de réalisation particulier de celle-ci, faite en référence aux dessins joints, donnés à titre d'exemples non limitatifs, et sur lesquels
- la figure 1 est une vue schématique de côté d'un dispositif de pince électro-chirurgicale selon un mode de réalisation avantageux de la présente invention ;
- la figure 2 est une vue de dessus du dispositif de la figure 1 ;
- la figure 3 est une vue de dessus agrandie de la partie de pince du dispositif des figures 1 et 2 ;
- la figure 4 est une vue en section transversale de la partie de poignée du dispositif de la figure 1, en position actionnée de la pince ;
- la figure 5 est une vue similaire à celle de la figure 4, en position actionnée du dispositif de coupe ;
- la figure 6 est une vue schématique en perspective de la pince en position fermée ;
- la figure 7 est une vue en section transversale de la pince en position fermée et la lame en position avancée ;
- la figure 8 est une vue similaire à la figure 6 en position ouverte de la pince ;
- la figure 9 est une vue en section transversale de la pince en position ouverte ;
- la figure 10 est une vue schématique en perspective du dispositif d'actionnement de la pince, en position ouverte ;
- la figure 11 est une vue similaire à celle de la figure 10 en position fermée de la pince ;
- la figure 12 est une vue de dessus du dispositif représenté sur les figures 10 et 11 ; et
- la figure 13 est une vue schématique en section transversale de la lame du dispositif de coupe.

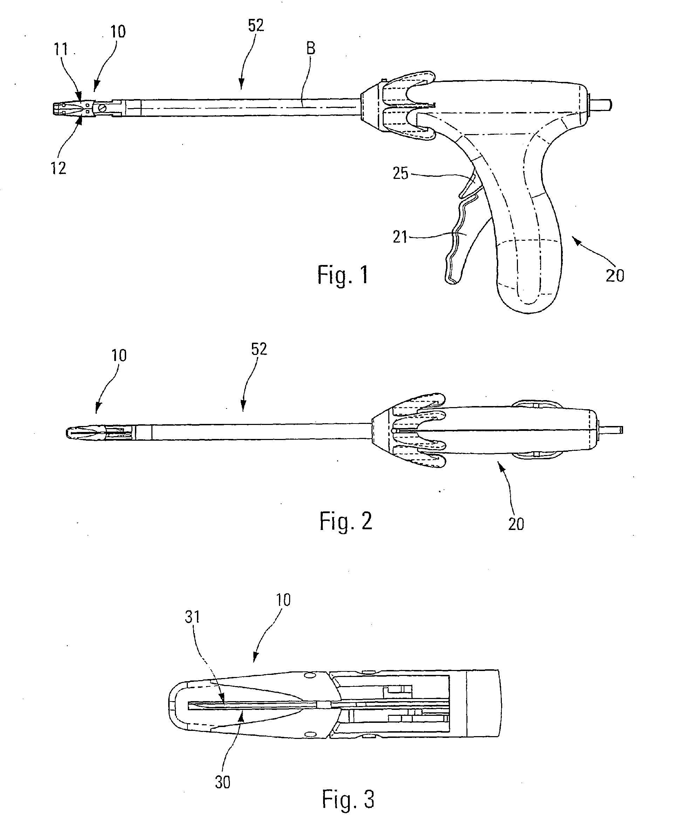

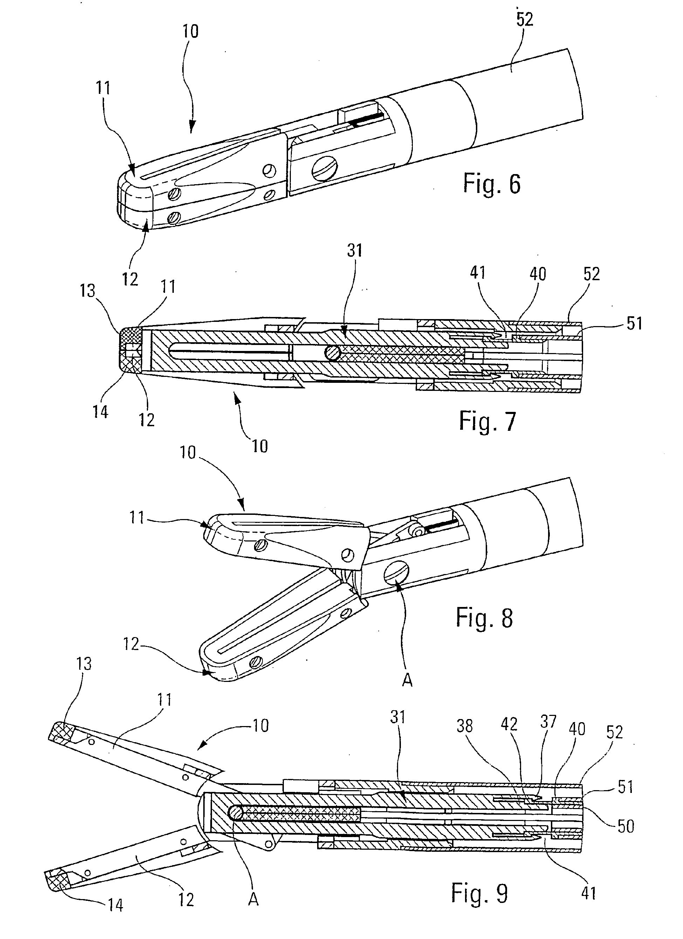

En se référant plus particulièrement aux figures 1 à 3, le dispositif de pince électro-chirurgicale de la présente invention comporte une poignée 20 reliée à une pince 10. La pince 10 comporte deux mors 11 et 12 déplaçables l'un par rapport à l'autre entre des positions ouverte et fermée de la pince 10. Des moyens de traitement, notamment de thermofusion, de tissus et/ou vaisseaux sont prévus, par exemple pour réaliser une thermofusion desdits tissus et/ou vaisseaux lorsque la pince est en position fermée. Le dispositif comporte aussi un dispositif de coupe 30, qui permet après traitement des tissus, de les découper directement au moyen du même dispositif. Ce dispositif de coupe 30 comporte une lame 31 déplaçable entre une position de repos rétractée dans la pince 10 (voir figure 9) et une position de coupe avancée dans la pince 10 (voir figure 7). La poignée 20 comporte un élément d'actionnement manuel 21 destiné à déplacer la pince 10 entre ses positions ouverte et fermée, ainsi qu'un élément de commande manuel 25 destiné à déplacer le dispositif de coupe 30, et donc la lame 31, entre ses positions rétractée et avancée. De préférence, la pince 10 peut en outre tourner librement autour de l'axe longitudinal B du tube (ou ensemble de fourreaux) qui relie la pince à la poignée 20, comme cela sera décrit plus en détail ci-après.

Le dispositif de la présente invention est donc adapté à la fois pour la chirurgie ouverte et pour la coeliochirurgie. Il est bien entendu que la forme particulière des différents éléments constitutifs du dispositif représenté sur les dessins n'est donnée qu'à titre indicatif et que ces divers éléments pourraient être réalisés avec une forme différente.

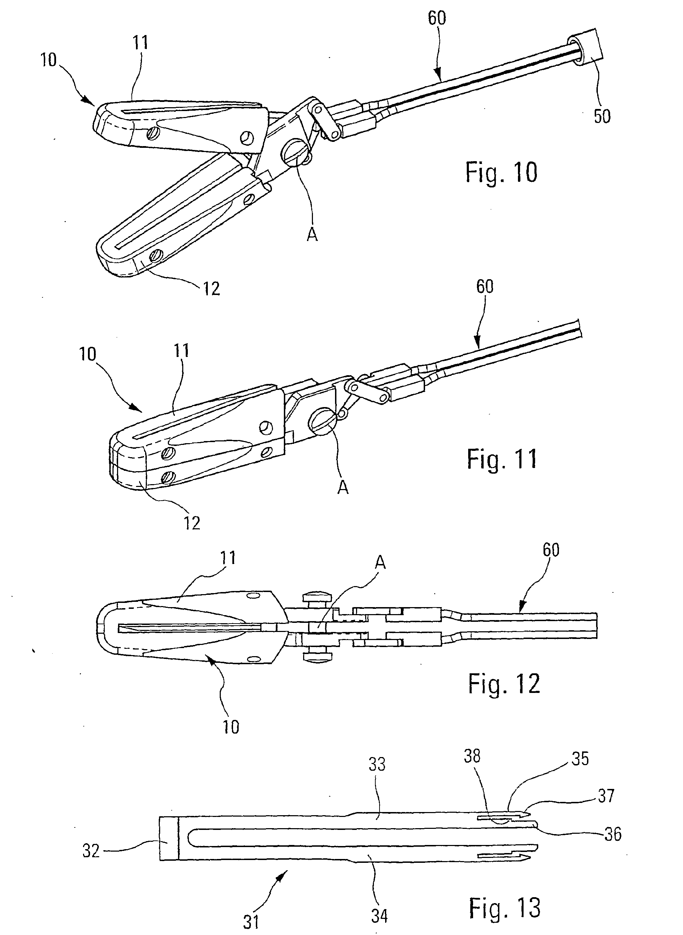

En se référant plus particulièrement aux figures 6 à 12, la pince 10 comporte deux mors 11, 12 déplaçables en rotation l'un par rapport à l'autre entre une position fermée de la pince 10 représentée sur les figures 1, 6, 7 et 11, et une position ouverte représentée sur les figures 8, 9 et 10. Avantageusement, les deux mors 11, 12 sont tous deux montés rotatifs autour d'un même axe de rotation A. Avantageusement, cet axe de rotation A est fixe par rapport à la poignée 20, c'est-à-dire que lorsque le chirurgien manipule la pince 10, entre ses positions ouvertes et fermées, ladite pince 10 n'avance ou ne recule pas à l'intérieur du corps du patient, mais reste au contraire à une distance fixe par rapport à la poignée. Ceci permet un positionnement beaucoup plus précis de la pince de thermofusion au moment où le chirurgien souhaite réaliser une thermofusion, et augmente donc la simplicité, la sécurité et la fiabilité d'utilisation de cette pince. Avantageusement, les deux mors 11, 12 sont chacun relié à une corde à piano respective 60 par l'intermédiaire de biellettes pivotantes, comme cela est représenté schématiquement sur les figures 10 et 11. Ces cordes à piano 60 sont de l'autre côté reliées à la poignée 20, et notamment à l'élément d'actionnement manuel de la pince 21, et peuvent être entourées par un premier fourreau 50, comme visible sur la figure 10.

De manière très avantageuse, la pince 10 de la présente invention est en position fermée lorsque l'élément d'actionnement manuel 21 de la pince est en position de repos. Ceci signifie que lorsque l'utilisateur ou le chirurgien introduit la pince à l'intérieur du corps du patient, les mors 11, 12 de la pince sont automatiquement en position fermée. Il peut donc tranquillement positionner avec précision la pince à l'intérieur du corps sans avoir à actionner simultanément la poignée, comme pour les dispositifs de l'art antérieur.

Avantageusement, la pince 10 peut tourner autour de l'axe longitudinal B de manière infinie dans les deux sens, c'est-à-dire qu'elle n'est pas bloquée à un moment donné dans sa rotation. Pour rendre l'utilisation de cette pince encore plus sûre, cette rotation peut être freinée lorsque la pince est en position fermée, c'est-à-dire lorsque l'élément d'actionnement manuel 21 est en position de repos. Ainsi, lorsque l'utilisateur introduit la pince à l'intérieur du corps du patient, la rotation longitudinale de la pince est freinée de sorte que le chirurgien peut très précisément positionner la pince à l'endroit souhaité sans risquer de voir celle-ci être tournée par les tissus environnants. Ce n'est que lorsque l'utilisateur actionne l'élément d'actionnement manuel 21 pour ouvrir la pince que ce frottement est diminué voire complètement supprimé permettant ainsi à ce moment là une rotation libre et infinie de la pince autour de l'axe longitudinal B.

Pour réaliser le traitement des tissus et/ou vaisseaux à traiter, en particulier la thermofusion, des moyens de transport d'énergie électrique sont prévus dans le dispositif. Ces moyens de transport peuvent comporter divers éléments conducteurs électriquement pour relier le générateur à chaque mors 11, 12. Lorsque l'énergie électrique est transmise aux mors par le générateur, les tissus et/ou vaisseaux à traiter se transforment en dégageant de la chaleur. Le courant électrique peut avantageusement être transmis par les cordes à piano 60 utilisées pour manipuler l'ouverture et la fermeture de la pince 10. En variante du mode de réalisation représenté sur les figures 10 à 12, où chaque mors est associé à une corde à piano respective, un dispositif d'ouverture et de fermeture de la pince 10 actionné par une seule corde à piano pourrait aussi être envisagé. Dans ce cas, les moyens de transmission du courant électrique vers les mors de la pince pourraient être réalisés par l'intermédiaire de fils électriques spécifiques. L'actionnement du traitement thermique peut être commandé par des moyens d'actionnement spécifiques (non représentés), tel qu'un bouton de commande, prévu sur la poignée 20. Lors d'une thermofusion, des puissances de 200 Watts avec une intensité de 4 ou 5 ampères sont par exemple envisageables, ce qui permet d'atteindre des températures élevées, de l'ordre de 200°C environ. En raison du passage d'un courant haute fréquence, et de la chaleur dissipée par les mors en position fermée lorsque la thermofusion se réalise, les surfaces externes des mors 11, 12 doivent être isolées électriquement et thermiquement. Ceci peut être réalisé par un revêtement, par exemple un gainage en une matière époxy ou autre ou par une céramique. Ce gainage peut toutefois présenter une certaine fragilité à la stérilisation. De préférence, l'invention prévoit donc des coiffes amovibles 13, 14, par exemple réalisées en PEEK, et qui peuvent être rapportées sur la surface externe de chaque mors 11, 12. Ces coiffes 13, 14 pourraient être collées, ou alors fixées par collage et/ou vissage ou goupillage. Un collage seul peut présenter des risques de faiblesse au niveau de la fixation, alors que l'utilisation du vissage ou du goupillage peut impliquer des difficultés de nettoyage. De préférence, la présente invention prévoit d'utiliser des coiffes 13, 14 amovibles à usage unique, ce qui facilite non seulement le nettoyage de la pince, mais évite aussi tout risque de détérioration dans le cadre d'une pince réutilisable et augmente donc la durée de vie de la pince. Par exemple, les coiffes pourraient être remplacées en même temps que la lame, avantageusement après chaque utilisation.

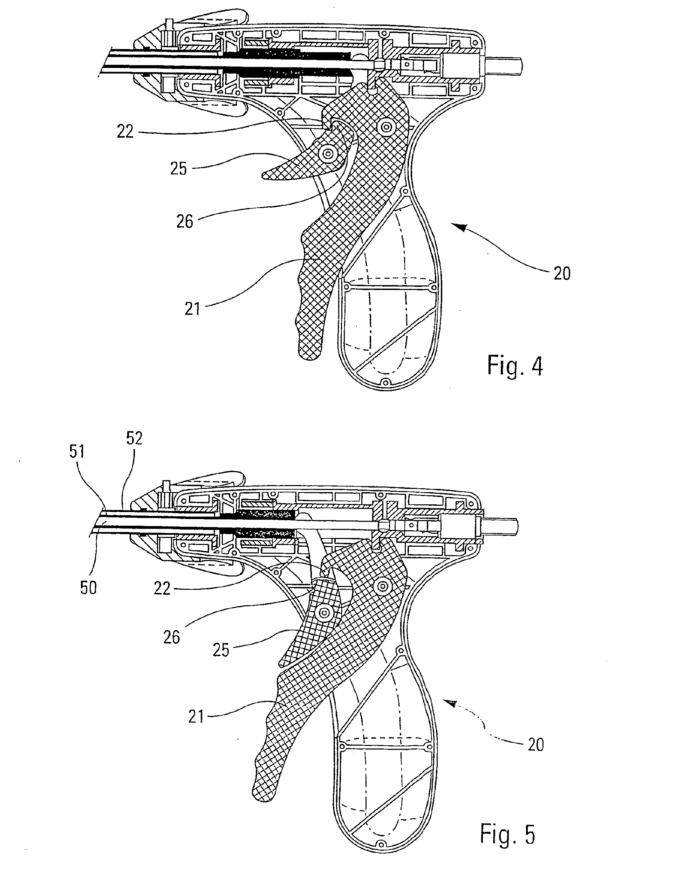

Le dispositif de l'invention comporte également un dispositif de coupe intégré 30. Ce dispositif de coupe 30 comporte une lame 31 dont l'extrémité avant 32 est pourvue de la partie coupante. Selon l'invention, ce dispositif de coupe 30 est démontable par rapport à la pince 10 et/ou par rapport à la poignée 20. Le fait de pouvoir démonter le dispositif de coupe 30 présente de nombreux avantages. D'une part, ceci permet de démonter et donc de nettoyer et décontaminer de manière efficace les différents constituants du dispositif, ainsi qu'une stérilisation de celui-ci, de sorte que la pince peut être réutilisée un nombre quelconque de fois. En effet, du fait de la présence du dispositif de coupe mobile, la pince lorsqu'elle est assemblée n'est pas totalement nettoyable et décontaminable, et ne peut donc ni être stérilisée, ni réutilisée. Le fait de pouvoir démonter le dispositif de coupe permet d'accéder aisément à tous les recoins de la pince, facilitant ainsi son nettoyage et sa décontamination. Avantageusement, le dispositif de coupe 30 comporte une lame 31 montée de manière amovible sur un élément de support 40, de sorte que ladite lame 31 pourrait être interchangée après chaque utilisation (ou après un nombre quelconque d'utilisations). Ceci garantit une qualité de coupe de la zone coupante 32 optimale à chaque utilisation en évitant d'utiliser une lame usée. Pour ce faire, la lame 31 a de préférence une forme en U avec chaque branche du U comportant à son extrémité libre des moyens de fixation de ladite lame 31 audit élément de support 40. Avantageusement, dans l'exemple représenté notamment sur la figure 13, chaque extrémité libre de la branche en U comporte deux projections axiales, une première projection 35, en l'occurrence la projection externe sur le dessin, étant pourvue d'un ergot d'encliquetage 37, et une seconde projection 36, la projection interne sur le dessin, comportant un épaulement de butée 38. Avantageusement, l'élément de support 40 comporte une gorge d'encliquetage 41 périphérique dans laquelle viennent s'encliqueter lesdits ergots d'encliquetage 37 de la lame 31. Cette position encliquetée est notamment visible sur la figure 9, dans laquelle on constate que la gorge d'encliquetage 41 de l'élément 40 est définie par une partie d'extrémité élargie 42 qui vient coopérer avec les épaulements de butée 38 pour définir la position de butée axiale de la lame 31 en position encliquetée. D'autre part, les ergots d'encliquetage 37 s'encliquètent derrière ladite partie d'extrémité 42, à l'intérieur de la gorge d'encliquetage 41 pour maintenir la lame fixée sur l'élément de support 40. De par la souplesse et l'élasticité de la lame, en particulier de la projection 35 qui supporte l'ergot d'encliquetage 37, une simple torsion de la lame 31 par rapport à l'élément de support 40 permet une séparation de la lame 31 de cet élément de support 40, et donc de manière très simple un nettoyage et/ou remplacement de celle-ci. Il est à noter que ces moyens de fixation amovibles de la lame 31 permettent au chirurgien de monter la lame en cours d'intervention chirurgicale en ouvrant simplement la pince 10 et en insérant la lame 31 qui vient alors automatiquement s'encliqueter dans la gorge d'encliquetage 41 de l'élément de support. De manière générale, le dispositif de l'invention est avantageusement démontable et remontable sans outils, ce qui facilite les opérations de nettoyage, décontamination et stérilisation après chaque utilisation, en vue de sa réutilisation ultérieure. Avantageusement, l'élément de support 40 est fixé et/ou solidaire d'un second fourreau 51 qui relie le dispositif de coupe 30 à la poignée 20, et notamment à l'élément de commande manuel 25 qui permet d'actionner le dispositif de coupe. Un déplacement axial de ce second fourreau 51 provoque donc le déplacement de la lame 31 entre ses positions rétractée et avancée. Dans l'exemple représenté, le dispositif de coupe 30 comporte la lame 31. En variante, l'élément de support 40 et/ou le second fourreau 51 (ou tout autre élément de liaison équivalent) pourraient aussi faire partie du dispositif de coupe 30, et ainsi selon l'invention être démontables par rapport à la pince 10 et/ou la poignée 20. Avantageusement, comme visible sur les figures 4 et 5, le dispositif de pince chirurgicale comporte des moyens de sécurité, et en particulier des moyens de blocage 22, 26 du dispositif de coupe 30, lesdits moyens de blocage étant actionnés lorsque les mors 11, 12 sont en position ouverte de la pince 10. Ceci garantit que le dispositif de coupe 30, notamment la lame 31, ne peut pas avancer à l'intérieur de la pince 10 lorsqu'elle est ouverte, ce qui pourrait occasionner des blessures à la fois à l'utilisateur mais aussi à l'intérieur du patient. De préférence, les moyens de blocage servent aussi à bloquer l'actionnement de la pince 10 lorsque la lame 31 est en position de coupe, c'est-à-dire avancée dans la pince. Ceci garantit que l'utilisateur ne peut pas ouvrir la pince 10 lorsque la lame est en position de coupe. De cette manière, la lame 31 n'est jamais dangereuse, ni en position rétractée, puisqu'alors elle n'est pas accessible, ni en position avancée, puisque celle-ci ne peut se produire que lorsque la pince est fermée, et qu'il est impossible d'ouvrir la pince lorsque la lame est avancée. Ces moyens de blocage, comme visible sur les figures 4 et 5, peuvent être formés par une projection de blocage 22 prévu sur l'élément d'actionnement manuel 21 qui sert à ouvrir et fermer la pince 10. En position actionnée de cet élément d'actionnement manuel 21, représentée sur la figure 4, l'ergot de blocage 22 coopère avec un épaulement 26 de l'élément de commande manuel 25 du dispositif de coupe 30 pour empêcher tout déplacement dudit élément de commande 25. Lorsque l'utilisateur relâche l'élément d'actionnement manuel 21 de la pince, celle-ci est ramenée automatiquement vers la position de repos représentée sur la figure 5, dans laquelle la projection de blocage 22 ne bloque plus l'actionnement de l'élément de commande 25. De ce fait, l'utilisateur peut utiliser le dispositif de coupe 30 seulement lorsque la pince 10 est en position fermée, comme visible sur la figure 5. De même, lorsque le dispositif de coupe 30 est en position de coupe (figure 5), la projection de blocage 22 repose contre la surface supérieure de l'épaulement 26. Ainsi, l'élément d'actionnement manuel 21 ne peut pas pivoter autour de son axe de pivotement, et il est donc bloqué en position de repos, c'est-à-dire avec la pince 10 en position fermée. Ce n'est que lorsque l'élément de commande 25 revient vers sa position de repos (avec la lame 31 rétractée), que la pince peut à nouveau être ouverte.

Comme évoqué précédemment, un avantage de la présente invention est que l'ensemble du dispositif peut être efficacement nettoyé puis stérilisé pour être réutilisé. Pour éviter d'avoir des zones difficiles ou impossibles à nettoyer, la lame 31 est avantageusement assemblée autour d'une gouttière réalisée dans un corps central disposé à l'intérieur de la pince. Ainsi, chaque branche du U de la lame 31 vient s'insérer dans une rainure supérieure respectivement inférieure dudit corps central de sorte que lorsque la lame est retirée, ces rainures qui sont ouvertes vers l'extérieur sont aisément accessibles pour être nettoyées. Avantageusement, la lame pourrait être réalisée en un matériau isolant, ou revêtue d'un matériau isolant. De cette manière, la lame pourrait être utilisée pour isoler un mors par rapport à l'autre, cette isolation étant nécessaire pour permettre une transmission de l'énergie électrique vers lesdits mors.

Avantageusement, la pince 10 est reliée à la poignée 20 par un ensemble de trois fourreaux concentriques. Un premier fourreau 50, qui est le fourreau radialement le plus à l'intérieur, entoure les cordes à piano 60 utilisées pour actionner la pince 10. Un second fourreau 51 disposé autour dudit premier fourreau 50 est adapté à supporter le dispositif de coupe 30 et est donc d'un côté relié à l'élément de commande manuel 25 et de l'autre à la lame 31. Enfin un troisième fourreau externe 52, disposé autour du second fourreau 51, est d'une part fixé à la pince, et d'autre part monté rotatif autour de la poignée 20.

La présente invention permet donc de fournir un dispositif de pince électro-chirurgicale utilisable en chirurgie ouverte et en coeliochirurgie, et qui présente notamment les caractéristiques avantageuses suivantes :
- le dispositif est applicable à la thermofusion.
- le dispositif est démontable.
- le dispositif est réutilisable, ce qui diminue le coût d'utilisation de celui-ci.
- le dispositif est facilement nettoyable, décontaminable et stérilisable.
- le dispositif présente une lame parfaitement affûtée à chaque utilisation.
- le dispositif simplifie l'utilisation par le chirurgien, notamment lors de l'insertion dans le corps, du fait que la pince est en position fermée lorsque la manette ou élément d'actionnement est en position de repos.
- le dispositif est démontable et remontable sans outils.

Bien que la présente invention a été décrite en référence à un mode de réalisation particulier de celle-ci, il est entendu que l'invention n'est pas limitée par ce mode de réalisation, mais qu'au contraire un homme du métier peut y apporter toutes modifications utiles sans sortir du cadre de la présente invention tel que défini par les revendications annexées.

## Revendications

1. Dispositif de pince électro-chirurgicale comportant une pince (10) et une poignée (20) manipulable par l'utilisateur, ladite pince (10) comportant deux mors (11, 12) déplaçables en rotation l'un par rapport à l'autre entre une position fermée et une position ouverte de la pince, le déplacement des mors (11, 12) étant commandé par un élément d'actionnement manuel (21) sur la poignée (20), ledit élément d'actionnement (21) étant déplaçable entre une position de repos et une position actionnée et étant élastiquement sollicité vers sa position de repos, ledit dispositif comportant des moyens de transfert d'énergie électrique dans la pince (10), pour réaliser un traitement, notamment une thermofusion, de tissus et/ou vaisseaux à traiter, et un dispositif de coupe (30) pour couper les tissus et/ou vaisseaux après traitement, le dispositif de coupe (30) est fixé de manière démontable à ladite pince (10) et/ou à ladite poignée (20), ledit dispositif de coupe (30) étant déplaçable entre une position de repos, dans laquelle le dispositif de coupe (30) est rétractée dans la pince, et une position de coupe, dans laquelle le dispositif de coupe (30) est avancé dans la pince pour couper des tissus et/ou vaisseaux, notamment après une thermofusion, le déplacement du dispositif de coupe (30) étant commandé manuellement par un élément de commande manuel (25) prévu sur la poignée (20), **caractérisé en ce que** lesdits mors (11, 12) sont en position fermée de la pince lorsque l'élément d'actionnement (21) est en position de repos.

2. Dispositif selon la revendication 1, dans lequel ledit dispositif de coupe (30) comporte une lame interchangeable (31).

3. Dispositif selon la revendication 2, dans lequel ledit dispositif de coupe (30) comporte un élément de support (40) recevant d'un côté ladite lame (31) et relié de l'autre côté à ladite poignée (20).

4. Dispositif selon la revendication 3, dans lequel ladite lame (31) a une forme sensiblement en U, l'extrémité libre de chaque branche (33, 34) du U comportant des moyens de fixation (35, 36, 37, 38) de la lame.

5. Dispositif selon la revendication 4, dans lequel chaque branche (33, 34) du U comporte à son extrémité libre deux projections (35, 36), une première projection (35) comportant un ergot d'encliquetage (37) et une seconde projection (36) comportant un épaulement de butée (38).

6. Dispositif selon la revendication 5, dans lequel ledit élément de support (40) comporte une gorge d'encliquetage (41) périphérique dans laquelle viennent s'encliqueter lesdits ergots d'encliquetage (37) de la lame (31).

7. Dispositif selon l'une quelconque des revendications 1 à 6, dans lequel la lame (31) est fixée sur l'élément de support (40) de manière amovible, ladite lame (31) étant séparée dudit élément de support (40) par torsion de la lame (31).

8. Dispositif selon l'une quelconque des revendications 2 à 7, dans lequel la lame (31) a une forme en U et s'assemble autour d'un corps central pourvu de deux rainures ouvertes vers l'extérieur, recevant chacune une branche dudit U.

9. Dispositif selon l'une quelconque des revendications 2 à 8, dans lequel ladite lame (31) est contenue dans et/ou revêtue d'un matériau élastiquement isolant.

10. Dispositif selon l'une quelconque des revendications précédentes, dans lequel lesdits mors (11, 12) sont montés en rotation l'un par rapport à l'autre autour d'un même axe de rotation (A) fixe par rapport à ladite poignée (20) en toutes positions desdits mors (11, 12).

11. Dispositif selon l'une quelconque des revendications précédentes, dans lequel, pour réaliser le traitement, tel qu'une thermofusion, des tissus et/ou vaisseaux à traiter, chaque mors (11, 12) reçoit de l'énergie électrique haute fréquence qu'il transmet auxdits tissus et/ou vaisseaux à traiter pour les transformer, la surface externe de chaque mors (11, 12) comportant des moyens d'isolation thermique et/ou électrique, tels qu'une couche de revêtement isolant et/ou une coiffe (13, 14) en matériau isolant.

12. Dispositif selon l'une quelconque des revendications précédentes, dans lequel lesdits mors (11, 12) sont assemblés de manière démontable l'un par rapport à l'autre.

13. Dispositif selon l'une quelconque des revendications précédentes, dans lequel le dispositif comporte des moyens de blocage (22, 26) dudit dispositif de coupe (30), lesdits moyens de blocage étant actionnés lorsque la pince (10) est en position ouverte.

14. Dispositif selon l'une quelconque des revendications précédentes, dans lequel le dispositif comporte des moyens de blocage (22, 26) de ladite pince (10), lesdits moyens de blocage étant actionnés lorsque le dispositif de coupe (30) est en position de coupe.

15. Dispositif selon les revendications 13 et 14, dans lequel lesdits moyens de blocage comportent une projection (22) solidaire de l'élément d'actionnement manuel (21) de la pince (10), ladite projection (22) coopérant avec une partie (26) de l'élément de commande manuel (25) du dispositif de coupe (30), pour d'une part bloquer le déplacement dudit élément de commande manuel (25) du dispositif de coupe lorsque ledit élément d'actionnement manuel (21) de la pince est en position actionnée, et pour d'autre part bloquer le déplacement dudit élément d'actionnement manuel (21) de la pince lorsque ledit élément de commande manuel (25) du dispositif de coupe est en position actionnée.

16. Dispositif selon l'une quelconque des revendications précédentes, dans lequel ladite pince (10) est reliée à ladite poignée (20) par au moins un fourreau (50, 51, 52) contenant des moyens de transmission d'actionnement de la pince (60), des moyens de transmission de courant électrique et des moyens de transmission d'actionnement du dispositif de coupe (30).

17. Dispositif selon la revendication 16, dans lequel les moyens de transmission d'actionnement de la pince comportent au moins une corde à piano (60) reliée d'une part auxdits mors (11, 12) et d'autre part à un élément d'actionnement manuel (21) de la poignée (20).

18. Dispositif selon la revendication 17, dans lequel ladite corde à piano (60) sert également de moyen de transmission de courant électrique.

19. Dispositif selon l'une quelconque des revendications 16 à 18, dans lequel ladite pince (10) est montée rotative autour de l'axe longitudinal (B) dudit au moins un fourreau (50,51, 52), ladite rotation étant infinie dans les deux sens.

20. Dispositif selon la revendication 19, dans lequel, en position fermée de la pince (10), ladite rotation de la pince (10) autour de l'axe longitudinal (B) est freinée par frottement, l'ouverture de la pince (10) diminuant et/ou supprimant sensiblement ledit frottement de freinage.

21. Dispositif selon l'une quelconque des revendications 16 à 20, dans lequel ledit dispositif comporte trois fourreaux concentriques (50, 51, 52), un premier fourreau (50) entourant les moyens de transmission d'actionnement de la pince (60), un second fourreau (51) supportant et/ou formant partie du dispositif de coupe (30), et un troisième fourreau (52) étant relié d'un côté à la pince (10) et de l'autre côté à la poignée (20).

22. Dispositif selon l'une quelconque des revendications précédentes, dans lequel le dispositif est nettoyable, décontaminable, stérilisable et réutilisable, le dispositif de coupe (30) et/ou la pince (10) étant interchangeables après un nombre quelconque d'utilisations.

23. Dispositif selon l'une quelconque des revendications précédentes, dans lequel ledit dispositif de pince est démontable et montable sans outils.

## Claims

1. Electro-surgical forceps device comprising forceps (10) and a handle (20) which can be operated by a user, the said forceps (10) comprising two jaws (11, 12) which can be moved in rotation relative to one another between a closed position and an open position of the forceps, the movement of the jaws (11, 12) being controlled by a manual actuating element (21) on the handle (20), the said actuating element (21) being able to be moved between a rest position and an actuating position and being elastically urged towards its rest position, the said device comprising means to transfer electric energy to the forceps (10) for performing treatment, in particular thermofusion of tissues and/or vessels to be treated, and a cutting device (30) for cutting tissues and/or vessels after treatment, the cutting device (30) is removably attached to the said forceps (10) and/or to the said handle (20), the said cutting device (30) able to be moved between a rest position in which the cutting device (30) is retracted inside the forceps, and a cutting position in which the cutting device (30) is moved forward in the forceps to cut tissue and/or vessels in particular after thermofusion, the movement of the cutting device (30) being controlled manually by a manual control element (25) provided on the handle (20), **characterized in that** the said jaws (11, 12) are in closed position of the forceps when the actuating element (21) is in rest position.

2. The device according to claim 1, wherein the said cutting device (30) comprises an interchangeable blade (31).

3. The device according to claim 2, wherein the said cutting device (30) comprises a supporting element (40) receiving the said blade (31) on one side and being connected on the other side to the said handle (20).

4. The device according to claim 3, wherein the said blade (31) is substantially U-shaped, the free end of each branch (33, 34) of the U comprising attaching means (35, 36, 37, 38) to attach the blade.

5. The device according to claim 4, wherein each branch (33, 34) of the U, at its free end, comprises two projections (35, 36), a first projection (35) comprising a press-fit lug (37) and a second projection (36) comprising an abutment shoulder (38).

6. The device according to claim 5 wherein the said supporting element (40) comprises a peripheral press-fit groove (41) into which the said press-fit lugs (37) of the blade (31) are press-fitted.

7. The device according to any of claims 1 to 6, wherein the blade (31) is removably attached onto the supporting element (40), the said blade (31) being separated from the said supporting element (40) by twisting the blade (31).

8. The device according to any of claims 2 to 7, wherein the blade (31) is U-shaped and is assembled around a central body provided with two outwardly opening grooves each receiving a branch of the said U.

9. The device according to any of claims 2 to 8, wherein the said blade (31) is contained in and/or coated with an elastically insulating material.

10. The device according to any of the preceding claims, wherein the said jaws (11, 21) are mounted in rotation relative to each other around one same rotation axis (A) that is fixed in relation to the said handle (20) at every position of the said jaws (11, 12).

11. The device according to any of the preceding claims wherein, to carry out treatment such as thermofusion of tissues and/or vessels to be treated, each jaw (11, 12) receives high frequency electric energy that it transmits to the said tissues and/or vessels to be treated for transforming thereof, the outer surface of each jaw (11, 12) comprising heat and/or electric insulating means such as a layer of insulating coating and/or capping (13, 14) in insulating material.

12. The device according to any of the preceding claims, wherein the said jaws (11, 12) are assembled so that they can be dismounted relative to each other.

13. The device according to any of the preceding claims, wherein the device comprises blocking means (22, 26) to block the said cutting device (30), the said blocking means being actuated when the forceps (10) are in open position.

14. The device according to any of the preceding claims, wherein the device comprises blocking means (22, 26) of the said forceps (10), the said blocking means being actuated when the cutting device (30) is in cutting position.

15. The device according to claims 13 and 14, wherein the said blocking means comprise a projection (22) secured to the manual actuating element (21) of the forceps (10), the said projection (22) cooperating with a part (26) of the manual control element (25) of the cutting device (30) so as firstly to block the movement of the said manual control element (25) of the cutting device when the said manual actuating element (21) of the forceps is in actuated position, and secondly to block the movement of the said manual actuating element (21) of the forceps when the said manual control element (25) of the cutting device is in actuated position.

16. The device according to any of the preceding claims, wherein the said forceps (10) are connected to the said handle (20) by at least one sheath (50, 51, 52) containing means for transmitting actuation of the forceps (60), means for transmitting electric current and means for transmitting actuation of the cutting device (30).

17. The device according to claim 16, wherein the means for transmitting actuation of the forceps comprise at least one piano wire (60) connected firstly to the said jaws (11, 12) and secondly to a manual actuating element (21) of the handle (20).

18. The device according to claim 17 wherein the said piano wire (60) also acts as means for transmitting electric current.

19. The device according to any of claims 16 to 18, wherein the said forceps (10) are mounted in rotation about the longitudinal axis (B) of the said at least one sheath (50, 51, 52), the said rotation being infinite in both directions.

20. The device according to claim 19 wherein, in the closed position of the forceps (10), the said rotation of the forceps (10) around the longitudinal axis (B) is checked by friction, the opening of the forceps (10) substantially reducing and/or cancelling the said frictional checking.

21. The device according to any of claims 16 to 20, wherein the said device comprises three concentric sheaths (50, 51, 52), a first sheath (50) surrounding the means for transmitting actuation of the forceps (60), a second sheath (51) supporting and/or forming part of the cutting device (30) and a third sheath (52) being connected on one side to the forceps (10) and on the other side to the handle (20).

22. The device according to any of the preceding claims, wherein the device can be cleaned, decontaminated, sterilized and re-used, the cutting device (30) and/or the forceps (10) being interchangeable after any number of uses.

23. The device according to any of the preceding claims wherein the said forceps device can be dismounted and mounted without tooling.

## Patentansprüche

1. Elektro-chirurgische Zangenvorrichtung mit einer Zange (10) und einem Griff (20), der durch den Benutzer bedienbar ist, wobei die Zange (10) zwei Backen (11, 12) aufweist, die zueinander drehbeweglich sind zwischen einer geschlossenen Stellung und einer geöffneten Stellung der Zange, wobei die Bewegung der Backen (11, 12) durch ein manuelles Betätigungselement (21) an dem Griff (20) gesteuert wird, wobei das Betätigungselement (21) zwischen einer Ruhestellung und einer betätigten Stellung beweglich ist und in Richtung seiner Ruhestellung federbelastet ist, wobei die Vorrichtung Mittel zur Übertragung elektrischer Energie in die Zange (10) aufweist, um eine Behandlung, insbesondere eine Thermofusion, von zu behandelndem Gewebe und/oder zu behandelnden Gefäßen, durchzuführen, und eine Schneidevorrichtung (30) aufweist, um das Gewebe und/oder die Gefäße nach der Behandlung zu schneiden, wobei die Schneidevorrichtung (30) an der Zange (10) und/oder an dem Griff (20) demontierbar befestigt ist, wobei die Schneidevorrichtung (30) beweglich ist zwischen einer Ruhestellung, in der die Schneidevorrichtung (30) in die Zange zurückgezogen ist, und einer Schneidestellung, in der die Schneidevorrichtung (30) in die Zange vorgeschoben ist, um das Gewebe und/oder die Gefäße, insbesondere nach einer Thermofusion, zu schneiden, wobei die Bewegung der Schneidevorrichtung (30) durch ein manuelles Steuerelement (25) manuell gesteuert wird, das an dem Griff (20) vorgesehen ist, **dadurch gekennzeichnet, dass** die Backen (11, 12) in geschlossener Stellung der Zange sind, wenn das Betätigungselement (21) in Ruhestellung ist.

2. Vorrichtung nach Anspruch 1, wobei die Schneidevorrichtung (30) eine auswechselbare Klinge (31) aufweist.

3. Vorrichtung nach Anspruch 2, wobei die Schneidevorrichtung (30) ein Trägerelement (40) aufweist, das auf der einen Seite die Klinge (31) aufnimmt und auf der anderen Seite mit dem Griff (20) verbunden ist.

4. Vorrichtung nach Anspruch 3, wobei die Klinge (31) im Wesentlichen eine U-Form hat, wobei das freie Ende jedes Schenkels (33, 34) des U Mittel zum Befestigen (35, 36, 37, 38) der Klinge aufweist.

5. Vorrichtung nach Anspruch 4, wobei jeder Schenkel (33, 34) des U an seinem freien Ende zwei Vorsprünge (35, 36) aufweist, wobei ein erster Vorsprung (35) eine Einrastnase (37) aufweist und ein zweiter Vorsprung (36) einen Anschlagabsatz (38) aufweist.

6. Vorrichtung nach Anspruch 5, wobei das Trägerelement (40) eine umlaufende Einrastrille (41) aufweist, in der die Einrastnasen (37) der Klinge (31) zum Einrasten kommen.

7. Vorrichtung nach einem der Ansprüche 1 bis 6, wobei die Klinge (31) an dem Trägerelement (40) auf lösbare Weise befestigt ist, wobei die Klinge (31) von dem Trägerelement (40) durch Verdrehung der Klinge (31) getrennt wird.

8. Vorrichtung nach einem der Ansprüche 2 bis 7, wobei die Klinge (31) eine U-Form hat und um einen mittigen Körper passt, der mit zwei Nuten versehen ist, die nach außen offen sind, die jeweils einen Schenkel des U aufnehmen.

9. Vorrichtung nach einem der Ansprüche 2 bis 8, wobei die Klinge (31) in einem elastisch isolierenden Material enthalten und/oder damit beschichtet ist.

10. Vorrichtung nach einem der vorhergehenden Ansprüche, wobei die Backen (11, 12) um ein und dieselbe Drehachse (A) herum relativ zueinander drehbar gelagert sind, die in allen Stellungen der Backen (11, 12) relativ zu dem Griff (20) ortsfest ist.

11. Vorrichtung nach einem der vorhergehenden Ansprüche, wobei, zur Durchführung der Behandlung, wie eine Thermofusion, des zu behandelnden Gewebes und/oder der zu behandelnden Gefäße, jede Backe (11, 12) elektrische Energie hoher Frequenz aufnimmt, die sie an das zu behandelnde Gewebe und/oder die zu behandelnden Gefäße überträgt, um sie umzuwandeln, wobei die äußere Fläche jeder Backe (11, 12) thermische und/oder elektrische Isoliermittel aufweist, wie eine Isolationsschicht und/oder eine Kappe (13, 14) aus Isoliermaterial.

12. Vorrichtung nach einem der vorhergehenden Ansprüche, wobei die Backen (11, 12) auf relativ zueinander demontierbare weise zusammengefügt sind.

13. Vorrichtung nach einem der vorhergehenden Ansprüche, wobei die Vorrichtung Mittel zum Sperren (22, 26) der Schneidevorrichtung (30) aufweist, wobei die Sperrmittel betätigt sind, wenn die Zange (10) in der geöffneten Stellung ist.

14. Vorrichtung nach einem der vorhergehenden Ansprüche, wobei die Vorrichtung Mittel zum Sperren (22, 26) der Zange (10) aufweist, wobei die Sperrmittel betätigt sind, wenn die Schneidevorrichtung (30) in der Schneidestellung ist.

15. Vorrichtung nach Anspruch 13 und 14, wobei die Sperrmittel einen Vorsprung (22) aufweisen, der mit dem Element für die manuelle Betätigung (21) der Zange (10) fest verbunden ist, wobei der Vorsprung (22) mit einem Teil (26) des Elements für die manuelle Steuerung (25) der Schneidevorrichtung (30) zusammenwirkt, um einerseits die Bewegung des Elements für die manuelle Steuerung (25) der Schneidevorrichtung zu sperren, wenn das Element für die manuelle Betätigung (21) der Zange in betätigter Stellung ist, und andererseits die Bewegung des Elements für die manuelle Betätigung (21) der Zange zu sperren, wenn das Element für die manuelle Steuerung (25) der Schneidevorrichtung in betätigter Stellung ist.

16. Vorrichtung nach einem der vorhergehenden Ansprüche, wobei die Zange (10) mit dem Griff (20) durch zumindest eine Griffhülse (50, 51, 52) verbunden ist, die Mittel für die Übertragung der Betätigung der Zange (10), Mittel für die Übertragung von elektrischem Strom und Mittel für die Übertragung der Betätigung der Schneidevorrichtung (30) umfasst.

17. Vorrichtung nach Anspruch 16, wobei die Mittel für die Übertragung der Betätigung der Zange zumindest einen Klaviersaitendraht (60) aufweisen, der einerseits mit den Backen (11, 12) und andererseits mit einem Element für die manuelle Betätigung (21) des Griffs (20) verbunden ist.

18. Vorrichtung nach Anspruch 17, wobei der Klaviersaitendraht (60) auch als Mittel für die Übertragung von elektrischem Strom dient.

19. Vorrichtung nach einem der Ansprüche 16 bis 18, wobei die Zange (10) um die Längsachse (B) der zumindest einen Griffhülse (50, 51, 52) herum drehbar gelagert ist, wobei die Drehung in beiden Richtungen unendlich ist.

20. Vorrichtung nach Anspruch 19, wobei, in geschlossener Stellung der Zange (10), die Drehung der Zange (10) um die Längsachse (B) herum durch Reibung gebremst wird, wobei die Öffnung der Zange (10) die Bremsreibung im Wesentlichen verringert und/oder unterdrückt.

21. Vorrichtung nach einem der Ansprüche 16 bis 20, wobei die Vorrichtung drei konzentrische Griffhülsen (50, 51, 52) aufweist, wobei eine erste Griffhülse (50) die Mittel für die Übertragung der Betätigung der Zange (10) umgibt, eine zweite Griffhülse (51) die Schneidevorrichtung (30) trägt und/oder einen Teil davon bildet und eine dritte Griffhülse (52) auf der einen Seite mit der Zange (10) und auf der anderen Seite mit dem Griff (20) verbunden ist.

22. Vorrichtung nach einem der vorhergehenden Ansprüche, wobei die Vorrichtung reinigbar, dekontaminierbar, sterilisierbar und wiederverwendbar ist, wobei die Schneidevorrichtung (30) und/oder die Zange (10) nach einer beliebigen Anzahl von Benutzungen auswechselbar sind.

23. Vorrichtung nach einem der vorhergehenden Ansprüche, wobei die Zangenvorrichtung ohne Werkzeug demontiert und montiert werden kann.
